# EUROPÄISCHE PATENTANMELDUNG

(11) **EP 0 750 047 A1**
(43) Veröffentlichungstag der Anmeldung: **27.12.1996**
(21) Anmeldenummer: 95109488.7
(22) Anmeldetag: 20.06.1995
(51) Int. Cl.: C12Q 1/68

(54) **Verwendung der Auftrennung von DNA-Fragmenten unter Anlagerung von polymergekoppelten DNA-Liganden zur Unterscheidung von Organismen, Viren, Plasmiden und Genen und zur Bestimmung ihrer Vielfalt**

(71) Anmelder: Meyer, Georg , Dr., D-28215 Bremen (DE); Rüggeberg, Hermann, Dr., D-28205 Bremen (DE)
(72) Erfinder: Meyer, Georg , Dr., D-28215 Bremen (DE); Rüggeberg, Hermann, Dr., D-28205 Bremen (DE)

(57) **Zusammenfassung**

Unterscheidung, Charakterisierung und/oder Bestimmung der Biodiversität von Organismen, Viren, Plasmiden, Genen oder Gruppen daraus (im folgenden 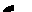 zu Identifizierenden") durch ligandengestützte Auftrennung von in ihnen enthaltenen DNA-Fragmenten oder von DNA-Fragmenten, die von ihrer RNA abgeleitet wurden. Fig. 2 zeigt ein einfaches Beispiel: (9), (10) und (11) zeigen die äquivalenten Fragmente eines Hydroxygenasegens aus drei unterschiedlichen Stämmen von Desulfovibrio in einem üblichen Agarosegel (8) und daneben in einem Gel, dem ein basensequenzspezifisch bindender polymergekoppelter DNA-Ligand zugesetzt wurde (13). (12) zeigt die Fragmente, die aus einer Mischkultur der drei Stämme vermehrt wurden. In dem üblichen Gel (8) sind die drei Stämme nicht zu unterscheiden. Die ligandengestützte Auftrennung (13) der Hydrogenasegenfragmente macht die Stämme dann unterscheidbar und zeigt, daß die Biodiversität in der Mischkultur (12) 'drei' ist.

## Beschreibung

### Beschreibung

Es handelt sich um eine Erfindung auf dem Gebiet der molekulargenetischen Differenzierung von Organismen, Viren, Plasmiden und Genen (im folgenden 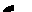 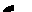 zu Identifizierenden").

Zur Unterscheidung und Identifizierung von zu Identifizierenden wird bereits die Darstellung und Unterscheidung von DNA-Fragmenten und DNA-Fragmentverteilungsmustern benutzt, die für die zu Identifizierenden typisch sind, die also im Unterschied zu anderen zu Identifizierenden nur bei den jeweils Untersuchten auftreten bzw. sich von deren Nukleinsäuren ableiten lassen.

Eine zur Zeit übliche Ausführungsform ist es, die DNA-Fragmente, deren Basenfolge für die zu Identifizierenden typisch ist oder die in Auftrennungsverfahren typische Verteilungsmuster ergeben, in der PCR-Reaktion (Polymerase chain reaction, Polymerase Kettenreaktion) zu vervielfältigen und anschließend die Länge einzelner Fragmente oder die Verteilungsmuster mehrerer Fragmente in gelelektrophoretischen Untersuchungen miteinander zu vergleichen. Da sich ein Unterschied in der Basenfolge häufig auch mit der Veränderung der Länge eines DNA-Fragments einhergeht, wird aus dem gelelektrophoretischen Vergleich der Länge oder des Verteilungsmusters der vermehrten Fragmente mit solchen Fragmenten, die aus Referenzorganismen, -viren, -plasmiden oder -genen vermehrt wurden, auf die Identität des/der untersuchten zu Identifizierenden bzw. auf deren Biodiversität geschlossen. Sind die Fragmente gleich lang oder ergeben sie ein gleiches Verteilungsmuster, wird davon ausgegangen, daß sie auch in der Sequenz gleich sind und daß die zu Identifizierenden, aus denen die Fragmente stammen, zu einer Gruppe gehören. Dies kann dann ausreichend sein, wenn bekannt ist, daß der Organismus nur einer kleinen und bereits bekannten Anzahl verschiedener zu Identifizierender angehören kann, deren Fragmentlängen bzw. Verteilungsmuster der typischen Sequenzen sich in vorher ermittelter Weise unterscheiden.

Die Bestimmung der Länge bzw. Längenzusammensetzung der vermehrten Fragmente reicht aber insbesondere in den Fällen nicht mehr zur Unterscheidung aus, in denen die Fragmente verschiedener zu Identifizierender längengleich oder in der Länge ähnlich sind, sich aber trotzdem in ihrer Basenfolge unterscheiden. Die zu Identifizierenden können dann nicht mehr in der oben beschriebenen Weise unterschieden werden.
Insbesondere in diesen Fällen ist es wichtig, die gleich langen organismentypischen Sequenzen zusätzlich auf die Identität ihrer Basenfolge zu überprüfen. Dazu ist die Sequenzierung (experimentelle Bestimmung der Basenfolge) der Fragmente die sicherste, gleichzeitig aber auch umständlichste und für die Untersuchung größerer Probenzahlen nicht geeignete Methode. Auch ist eine Sequenzierung unmöglich, solange die in ihrer Basenfolge unterschiedlichen Sequenzen wegen ihrer gleichen Länge nicht getrennt voneinander dargestellt werden können.

Es ist also ein Schritt in die Untersuchung einzuführen, der gleich lange, in ihrer Basenfolge aber unterschiedliche Sequenzen voneinander unterscheidbar macht, ohne sie sequenzieren zu müssen.

Zu einer solchen Trennung kann man die mit einer Veränderung der Basenfolge in der Regel verbundenen Änderungen physikalischer Parameter ausnutzen.

Es sind bereits verschiedene hierauf basierende sogenannte DNA-Screening-Methoden beschrieben (E.P. Lessa und G. Applebaum, Screening techniques for detecting allelic variation in DNA sequences, Molecular Ecology 2, 119-129, 1993), die DNA-Fragmente gleicher Länge aber unterschiedlicher Basenzusammensetzung in der Polyacrylamidgelelektrophorese auftrennen.
Einzelsträngige DNA-Moleküle mit unterschiedlicher Basenfolge können im 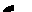 SSCP"-Verfahren (single stranded conformation polymorphism) aufgetrennt werden. Die Trennung beruht hier auf der unterschiedlichen Konformation der Moleküle. Doppelsträngige DNA-Moleküle lassen sich durch die 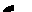 DGGE" (denaturing gradient gel electrophoresis sowie die TGGE (temperature gradient gel electrophoresis) auftrennen. Hier beruht die Trennung auf der Sequenzabhängigkeit des Schmelzverhaltens.
Für die Durchführung dieser Methoden sind teure und spezielle Ausrüstungen notwendig. Der Anpassungaufwand der Verfahren für individuelle Fragestellungen ist hoch. Sowohl die Auftrennungen selbst als auch anschließende weitergehende Untersuchungen der aufgetrennten Nukleinsäuren wie durch Hybridisierung (Identifizierung durch Paarung mit komplementären DNA-Sequenzen) oder Sequenzierung (Bestimmung der Basenfolge) sind zeitaufwendig.
Die wichtigste Einschränkung der oben genannten Methoden besteht darin, daß die optimale Auftrennung der DNA-Fragmente auf einen Längenbereich von bis circa 350 Basenpaaren im SSCP-Verfahren bzw. bis circa 500 Basenpaaren in den DGGE-und TGGE-Verfahren beschränkt ist. Damit ist auch die Information, die man nach einer der Auftrennung gegebenenfalls folgenden Sequenzierung der aufgetrennten DNA-Fragmente erhält, auf kurze Fragmentlängen beschränkt. Eine Auftrennung längerer Fragmente, z.B. ganzer Gene, zur Charakterisierung von zu Identifizierenden ist unmöglich.
Über die Polyacrylamidgelelektrophorese hinaus besteht auch die Möglichkeit, die DNA-Fragmente in der Dichtezentrifugation aufzutrennen (Owen, R.J. and Hill, L.R., in: Identification Methods for Microorganisms 1979, Latimer Trend & Company LTD, Plymouth, S. 281-285). Die Dichtezentrifugation nutzt die sich in Abhängigkeit von der Basenzusammensetzung ändernde Dichte der Sequenzen als Trennparameter. Sie setzt aber einen hohen Aufwand an Chemikalien und Maschinen voraus und hat den Nachteil, daß zur Auftrennung große Mengen der aufzutrennenden Sequenzen vorliegen müssen.

Die Erfindung vereinfacht die Unterscheidung von zu Identifizierenden durch die Verwendung eines anderen und wesentlich einfacheren Verfahrens zur getrennten Darstellung von DNA-Fragmenten, die sich in ihrer Basenfolge unterscheiden. Als diskriminierender Parameter wird hier das unterschiedliche Bindungsverhalten der aufzutrennenden Fragmente an basensequenzspezifisch bindende Liganden benutzt, die mit langen Polymerketten verbunden sind (Müller, W., Hattesohl, I., Schütz, H.-J. und Meyer, G., 1981, Nucleic Acids Research 9, 95-119). In der Gelelektrophorese erhöhen die polymergekoppelten Liganden die Reibungskoeffizienten der aufzutrennenden DNA-Fragmente basensequenzspezifisch. Dies führt zu einer wiederum basensequenzabhängigien Verzögerung der Laufgeschwindigkeit und damit zur Auftrennung der individuellen DNA-Fragmente.
Das Prinzip wurde nach der Veröffentlichung lediglich in einigen Fällen in der Grundlagenforschung zur Klärung der organismeninternen Struktur und Funktion der DNA genutzt (Kruse, L., Meyer, G., und Hildebrandt, A., 1993, Biochimica et Biophysica Acta, 1216, 129-133). Die Potenz der Anwendung im hier dargestellten Fragenkreis wurde bisher trotz der weltweiten Suche nach einfachen Verfahren zur Verfeinerung der Auftrennung von DNA-Fragmenten zum Zwecke der Unterscheidung von zu Identifizierenden als auch der damit verbundenen Bestimmung ihrer Vielfalt nicht erkannt.

Gemäß Patentanspruch 1 werden die zu Identifizierenden hier unterschieden, indem ihre typischen DNA-Fragmente, deren Längen bzw. Längenverteilung in gelelektrophoretischen Verfahren identisch sein kann, unter Verwendung der Anlagerung von Ligandenpolymeren aufgetrennt werden. Durch Vergleich mit Standards kann dann auf die Identität der zu Identifizierenden und auf deren Vielfalt (Biodiversität) in einer Probe geschlossen werden.
Für die Untersuchungen macht es keinen Unterschied, ob die aufzutrennenden DNA-Fragmente direkt aus den Nukleinsäuren der zu Identifizierenden gewonnen werden oder ob sie von RNA-Sequenzen abgeleitet werden, die bzw. deren Verteilung für die zu Identifizierenden typisch sind (Patentanspruch 1) und, ob sie oder Teile davon in einem Zwischenschritt in Verfahren wie zum Beispiel der PCR oder der Klonierung (Patentanspruch 2) vermehrt wurden.

Eine zur Zeit vorteilhafte Ausgestaltung der Erfindung ist im Patentanspruch 2 angegeben. Die DNA der zu Identifizierenden wird isoliert. Die für die jeweiligen zu Identifizierenden typischen DNA-Fragmente werden in der PCR vermehrt. Stellen sich die Fragmente verschiedener zu Identifizierender oder verschiedener Populationen zu Identifizierender in herkömmlichen Agarose- oder Polyacrylamidgelen als gleich lang bzw. gleich verteilt dar, können sie durch erneuten Auftrag in einem folgenden Agarosegel, dem ein basensequenzspezifischer, polymergebundener Ligand zugesetzt wurde, einfach darauf überprüft werden, ob sie auch bezüglich ihrer Basenzusammensetzung identisch sind.
Der erneute Auftrag kann auch ersetzt werden, indem die in herkömmlichen Gelen nach der Länge aufgetrennten DNA-Fragmente mit der sie umgebenden Gelmatrix ausgeschnitten und vertikal zur ersten Laufrichtung in ein neues Gel eingegossen werden, das einen polymergebundenen DNA-Liganden enthält und die einzelnen Banden entsprechend der Länge und Basenzusammensetzung auftrennt. Entsprechend kann auch in einem ersten Lauf nach Länge und Basenzusammensetzung und in dem folgenden rein nach der Länge aufgetrennt werden.

Die mit der Erfindung einhergehenden Vorteile bestehen darin, daß insbesondere solche zu Identifizierende, die aufgrund der identischen Längen der zu ihrer Unterscheidung untersuchten DNA-Fragmente in erster Näherung für identisch gehalten werden, wesentlich einfacher als bisher voneinander unterschieden werden können. Im Vergleich mit SSCP, DGGE und TGGE ist auch von Vorteil, daß hier Agarosegele verwendet werden können. Agarosegele sind wesentlich einfacher zu handhaben als Polyacrylamidgele und ihr Einsatz geht nicht mit der Verwendung toxischer Substanzen einher. Es können DNA-Fragmente im Längenbereich von mehreren Tausend Basenpaaren aufgetrennt werden. Darüberhinaus werden die untersuchten Sequenzen auf einfache Weise weiteren Untersuchungen wie der Hybridisierung und der Sequenzierung zugänglich, die auch die Bestimmung durch den Abgleich mit Sequenzdatenbanken und die Ableitung von DNA-Sonden und Oligonukleotiden ermöglichen. Sowohl die Bindung an Hybridisierungsmembranen als auch die Elution der dargestellten Fragmente sind vergleichsweise sehr einfach. Bei dem Einsatz von DNA-Fragmenten aus gemischten Populationen unbekannter Zusammensetzung zu Identifizierender lassen sich neben der erleichterten Identifizierung der zu Identifizierenden auch vereinfacht Rückschlüsse auf die Organismenvielfalt (Biodiversität) ziehen. Die Identifizierung nicht anzüchtbarer bislang nicht identifizierbarer Mikroorganismen z.B. durch Charakterisierung ihrer PCR-Fragmente wird präziser.
Im Bereich der Untersuchung von Krankheitskeimen eröffnet die Erfindung Perspektiven auch im Bereich der Feindifferenzierung von Erregern in medizinischen Analyselabors, so zum Beispiel bei der Verfolgung von Infektionswegen, der Zuordnung von Gefahrenpotentialen, der Entlarvung von krankenhaustypischen Keimen, Viren und Plasmiden.

Insbesondere in Fällen, in denen sich herausstellt, daß Fragmente, die sich in herkömmlichen Gelen identisch verhalten, aufgetrennt werden können, eröffnet sich eine einfache Perspektive der Verwendung der Fragmente zur Ableitung von DNA-Sonden oder Oligonukleotiden (Patentanspruch 3).

Werden Fragmentgemische aufgetrennt, kann deren Muster entweder direkt oder nach einer Hybridisierung als individuen- oder gruppentypischer 'Fingerprint' verwendet werden (Patentanspruch 4).

Handelt es sich bei den aufgetrennten Fragmenten um Gene oder Teil davon können die zugehörigen zu Identifizierenden bezüglich ihrer Eigenschaften wie zum Beispiel Krankheit, Pathogenität, biochemischer Parameter charakterisiert werden (Patentanspruch 5).

Im folgenden soll das Prinzip der Erfindung anhand von zwei einfachen Ausführungsbeispielen unter Bezugnahme auf die beigefügten Abbildungen erläutert werden. Dabei zeigt:

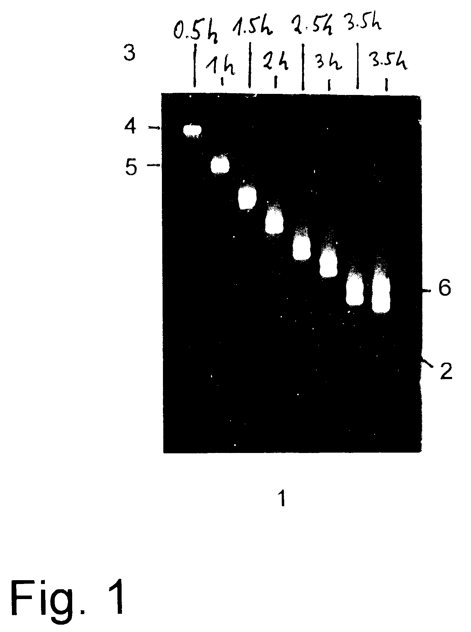

### Beispiel 1 (Fig. 1)

Mit der Laufzeit zunehmende Unterscheidbarkeit von Desulfovibrio baculatus und Desulfovibrio gigas durch die Auftrennung ihrer ca. 440 Basenpaare langen organismentypischen Fragmente des Hydrogenasegens in der Gelelektrophorese mit einem polymergekoppelten basensequenzspezifischen DNA-Liganden, der sich bevorzugt an adenin- und thymidinreiche DNA-Bereiche anlagert.

PCR-Fragmente (2) entsprechend der Region Hyd 1/5 zwischen Pos. 1441 bis 1879 des (NiFe)Hydrogenasegens von Desulfovibrio vulgaris (Wawer, C. und Muyzer, G., 1995, Appl. Environm. Biol. 61, 2203-2210) aus einer Bakterienkultur, die sich in herkömmlichen Gelen als längengleich darstellen, sind in Zeitabständen mit den resultierenden Laufzeiten (3), Angabe in Stunden (h) in einem Agarosegel (1) aufgetragen, das unmittelbar vor dem Gießen mit 0.025 OD₃₄₀/ml (OD₃₄₀= optische Dichte bei 340 nm, ml= Milliliter) des an Polyäthylenglycoll 6000 gebundenen DNA-Liganden Bisbenzimid pro ml Gellösung versetzt wurde. Agarosekonzentration: 2%, Puffer: 25 mM EDTA, pH: 5,9, Spannung 2 V/cm. Anfärbung der DNA mit Ethidiumbromid.

Die DNA-Fragmente laufen zunächst als eine Bande in das Gel hinein (4), als stammen sie sämtlich aus identischen Organismen. Bereits nach einer Stunde wird aber erkennbar, daß die Probe zwei unterschiedliche Organismen enthalten muß (5). Durch den Vergleich der Laufstecken der Fragmente mit denen der daneben aufgetragenenFragmente aus Referenzorganismen (6), von oben nach unten Desulfovibrio desulfuricans, D. baculatus, D. gigas und D. vulgaris, stellt sich heraus, daß die Kultur aus Desulfovibrio gigas und Desulfovibrio baculatus besteht. Das Fragmentmuster kann durch Southern Blot nach den Standardprotokollen für Agarosegele an einen Filter gebunden und so leicht hybridisiert werden. Auch können die Fragmente nach der Trennung einfach aus dem Gel eluiert werden, um davon DNA-Sonden/Oligonukleotide abzuleiten und/oder sie zu sequenzieren.

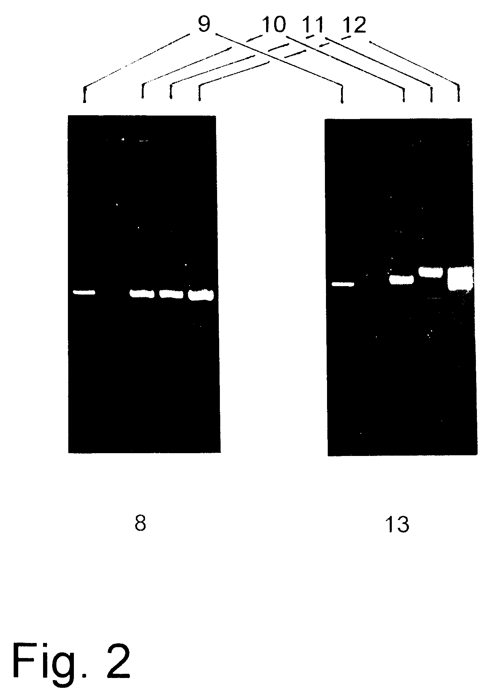

### Beispiel 2 (Fig. 2)

die Unterscheidung und Identifizierung nah verwandter Desulfurikanten aus einem Gemisch, deren typische Fragmente wegen ihrer identischen Länge in herkömmlichen Gelen den Schluß nahelegen würden, es handle sich nur um eine anstatt tatsächlich drei Sorten von Desulfurikanten und die Feststellung ihrer Biodiversität durch die Auftrennung ihrer ca. 1400 Basenpaare langen organismentypischen Fragmente des Hydrogenasegens in der Gelelektrophorese mit einem polymergekoppelten DNA-Liganden. Die Nichtunterscheidbarkeit der Organismen in der herkömmlichen Gelelektrophorese ist der Unterscheidbarkeit in der Agarosegelelektrophorese mit dem Zusatz eines polymergekoppelten basensequenzspezifisch bindenden DNA-Liganden gegenübergestellt.

Die organismentypischen DNA-Fragmente von Desulfovibrio longus (9), D. gigas (19) und D. desulfuricans (11) und aus einem Gemisch der drei Bakterien (12) entsprechend der Region Hyd 1/7 zwischen Pos. 1441 bis 2878 des (NiFe)Hydrogenasegens von Desulfovibrio vulgaris (Deckers, H.H., Wilson, F.P. und Voordow, 1990, J. Gen. Microbiol. 136, 2021-2028) wurden in der PCR vervielfältigt und in einem herkömmlichen Agarosegel (8), Agarosekonzentration: 1.5 %, Puffer: 25 mM EDTA, pH 5,9 bei 3 V/cm aufgetrennt. Anfärbung der DNA mit Ethidiumbromid. Die Fragmente weisen die gleiche Länge auf und stellen sich insoweit als aus identischen Organismen stammend dar. Die Position der Fragmente aus den drei Stämmen legt fälschlicherweise die Identität der drei Organismen nahe.

Die gleichen vervielfältigten Fragmente wurden anschließend in einem zweiten Agarosegel (13) aufgetrennt, dem unmittelbar vor dem Gießen 0.025 OD₃₄₀ (OD₃₄₀= optische Dichte bei 340 nm) des an Polyäthylenglycoll 6000 gebundenen DNA-Liganden Bisbenzimid pro ml Gellösung zugesetzt wurde. Agarosekonzentration: 2%, Puffer: 25 mM EDTA, pH 5.9, Spannung: 2 Volt/cm.

Das zweite Gel (13) zeigt, daß die drei verschiedenen Bakterienarten aufgrund der unterschiedlichen Basenfolge ihrer gleich langen typischen DNA-Fragmente durch den Zusatz des polymergekoppelten Liganden zu den Gelen unterschieden werden können. Die im Vergleich zu den in der Polyacrylamidgelelektrophorese auftrennbaren Fragmenten sehr große Länge der Fragmente behindert die Unterscheidung der Organismen nicht.
Die Anzahl der Fagmente im Gel entspricht der Biodiversität (organismischen Vielfalt) in der Probe. Die Biodiversität beträgt hier: drei (s. (12) im zweiten Gel (13)).

## Patentansprüche

1. Unterscheidung, Charakterisierung und/oder Bestimmung der Biodiversität von Organismen, Viren, Plasmiden, Genen oder Gruppen daraus (im folgenden 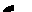 zu Identifizierenden") durch ligandengestützte Auftrennung von in ihnen enthaltenen DNA-Fragmenten oder von DNA-Fragmenten, die von ihrer RNA abgeleitet wurden.

2. Verwendung nach Anspruch 1,
dadurch gekennzeichnet, daß zu Identifizierende durch die getrennte Darstellung und Unterscheidung von DNA-Fragmenten, die in Vermehrungsverfahren wie der PCR oder die Klonierung vermehrt wurden, unterschieden und charakterisiert werden und/oder hierdurch ihre Biodiversität bestimmt wird.

3. Verwendung nach einem der Ansprüche 1 bis 2,
dadurch gekennzeichnet, daß von den aufgetrennten Fragmenten DNA-Sonden oder Oligonukleotide abgeleitet werden.

4. Verwendung nach einem der Ansprüche 1 bis 2,
dadurch gekennzeichnet, daß bei der Auftrennung entstehende DNA-Fragmentmuster zur Unterscheidung, Charakterisierung und/oder Bestimmung der Biodiversität von zu Identifizierenden als DNA-Fingerprints oder zur Anfertigung von DNA-Fingerprints benutzt werden.

5. Verwendung nach einem der Ansprüche 1 bis 2,
dadurch gekennzeichnet, daß durch Untersuchung von Genen oder Genfragmenten Eigenschaften und Zugehörigkeiten der zu Identifizierenden ermittelt werden.
